# EUROPÄISCHE PATENTANMELDUNG

(11) **EP 2 433 575 A1**
(43) Veröffentlichungstag der Anmeldung: **28.03.2012**
(21) Anmeldenummer: 11181385.3
(22) Anmeldetag: 15.09.2011
(51) Int. Cl.: A61B 17/16

(54) **Medizinischer Bohrer**

(30) Priorität: 23.09.2010 DE 102010046419
(71) Anmelder: Richard Wolf GmbH, 75438 Knittlingen (DE)
(72) Erfinder: Herdrich, Christoph, 79369 Wyhl am Kaiserstuhl (DE); Körner, Eberhard, 75438 Knittlingen (DE); Berg, Peter, 76703 Kraichtal (DE)
(74) Vertreter: Hemmer, Arnd

(57) **Zusammenfassung**

Ein medizinischer Bohrer weist einen Schaft auf. Im Bereich eines distalen Endes dieses Schafts ist ein Schneidkörper feststehend angeordnet, der radial an dem Schaft auskragt und der eine einem proximalen Ende des Schaftes zugewandte Schneide aufweist.

## Beschreibung

Die Erfindung betrifft einen medizinischen Bohrer.

In der Orthopädie sind Operationstechniken üblich, bei denen künstliche Knochenkanäle gebohrt werden müssen, die an einem Endbereich einen gegenüber dem übrigen Kanal größeren Querschnitt aufweisen. Solche Knochenkanäle sind beispielsweise bei verschiedenen Operationsmethoden zur Rekonstruktion von Ligamenten erforderlich. Hier dient der erweiterte Bereich des Knochenkanals zur Aufnahme eines Endes eines Ligament-Transplantats, das mittels einem an dem Transplantat befestigten Faden, der durch den schmaleren Abschnitt des Knochenkanals geführt ist, außerhalb des Kanals an dem Knochen festgelegt ist.

Die Bohrung von abgestuften künstlichen Knochenkanälen erweist sich insbesondere bei einer Rekonstruktion des vorderen Kreuzbandes als problematisch, da in diesem Fall die erweiterten Bereiche der zum Festlegen des Transplantats erforderlichen Knochenkanäle an Schienbein und Oberschenkel an den Gelenkspalt des Kniekehlgelenks angrenzend ausgebildet werden müssen.

Zu diesem Zweck dient ein aus EP 1 785 103 A1 bekanntes System zum retrograden Bohren von Knochen. Hier wird mit einem Bohrer zunächst ein in dem Kniekehlgelenk mündender Knochenkanal gebohrt und ein zusätzlicher in dem Gelenkspalt des Kniekehlgelenks mündender Kanal geschaffen, über den an dem in dem Gelenkspalt befindlichen Bohrerende ein Bohraufsatz montiert wird. Danach wird mit dem Bohraufsatz bei einer Rückwärtsbewegung des Bohrers aus der Bohrung der an das Kniekehlgelenk angrenzende erweiterte Bereich des Knochenkanals aufgebohrt. Anschließend muss der Bohraufsatz von dem Bohrer demontiert werden und aus dem Gelenkspalt des Kniekehlgelenks entfernt werden.

Eine weniger aufwändige Vorgehensweise ermöglicht ein aus EP 2 098 177 A1 bekannter Bohrer, der an seinem distalen Ende eine Zusatzschneide aufweist, die aus dem Bohrerschaft radial ausklappbar ist. Mit diesem Bohrer wird zunächst bei einer in dem Schaft eingeklappten Schneide ein in dem Kniekehlgelenk mündender Knochenkanal geschaffen, dessen an das Gelenk angrenzender Bereich mit der ausgeklappten Zusatzschneide durch eine rückwärts gerichtete Bohrbewegung des Bohrers erweitert wird. Aufgrund der bewegbaren Zusatzschneide weist der Bohrer eine vergleichsweise komplizierte Mechanik zum Auf- und Einklappen der Zusatzschneide und eine verhältnismäßig geringe Stabilität auf.

Vor diesem Hintergrund liegt der Erfindung die Aufgabe zugrunde, einen medizinischen Bohrer zur Verfügung zu stellen, der bei einem einfachen Aufbau und einfacher Handhabung insbesondere bei der Rekonstruktion des vorderen Kreuzbandes die Schaffung von Stufenbohrungen ermöglicht.

Gelöst wird diese Aufgabe durch einen medizinischen Bohrer mit den in Anspruch 1 angegebenen Merkmalen. Vorteilhafte Weiterbildungen dieses Bohrers ergeben sich aus den Unteransprüchen, der nachfolgenden Beschreibung sowie den Zeichnungen. Hierbei können gemäß der Erfindung die in den Unteransprüchen angegebenen Merkmale jeweils für sich aber auch in technologisch sinnvoller Kombination die erfindungsgemäße Lösung gemäß Anspruch 1 weiter ausgestalten. Der erfindungsgemäße Bohrer weist einen Schaft auf, an dem im Bereich seines distalen Endes ein an dem Schaft radial über den Außenumfang des Schaftes auskragender Schneidkörper angeordnet ist. Die Anordnung des Schneidkörpers an dem Schaft ist feststehend, so dass der Schneidkörper mit dem Schaft eine starre Einheit bildet. D.h., der Schaft ist nicht einklappbar. Der Schneidkörper weist an seinem auskragenden Bereich eine Schneide auf, die dem proximalen Ende des Schafts zugewandt ist. D.h., die an dem Außenumfang des Schafts radial auskragende Schneide bzw. Schneidkante des Schneidkörpers weist in Richtung des proximalen Bohrerendes, das in eine Bohrmaschine eingespannt werden kann.

Mit diesem vergleichsweise einfachen Aufbau ermöglicht der erfindungsgemäße medizinische Bohrer die schnelle und einfache Schaffung von abgestuften Knochenkanälen mit einer retrograden Erweiterung der Knochenkanäle an der von dem Bohreransatz abgewandten Knochenseite, wie sie bei vielen Operationstechniken im Bereich der Orthopädie erforderlich sind. Hierzu wird der Bohrer zum Einbringen in den Teil des Knochenkanals mit geringerem Durchmesser nicht angetrieben, d.h. ohne Bohrerdrehung ähnlich der Verwendung eines Meißels durch den Knochen getrieben, bis der distale Endbereich des Bohrers mit dem daran angeordneten Schneidkörper an der gegenüberliegenden Seite des Knochens aus diesem herausragt. Anschließend wird der Bohrer unter üblicher Bohrerdrehung zurückbewegt und auf diese Weise mittels des Schneidkörpers, dessen Schneide dann dem Knochen zugewandt ist, ein erweiterter Bereich des zu schaffenden Knochenkanals retrograd aufgebohrt.

Zweckmäßigerweise kann vor dem Durchstemmen des Knochens mit dem erfindungsgemäßen Bohrer mit einem gewöhnlichen zylindrischen Bohrer eine Vorbohrung durch den Knochen vorgenommen werden, deren Durchmesser dem kleineren Durchmesser des zu schaffenden Knochenkanals in dessen nicht erweiterten Abschnitt entspricht. Hierdurch wird das Durchstemmen des Knochens erleichtert, da nur von dem gegenüber dem Bohrerschaft radial vorspringenden Schneidkörper Knochengewebe unter Bildung einer Nut abgetragen werden muss. Insofern bildet der erfindungsgemäße Bohrer quasi auch ein Räumwerkzeug und der Schneidkörper eine zusätzliche Schneide zum Räumen der Nut. Nach dem Aufbohren des erweiterten Abschnitts des Knochenkanals wird der erfindungsgemäße Bohrer zweckmäßigerweise so gedreht, dass sich der Schneidkörper in Flucht mit dem Lichtraumprofil der Nut befindet und in dieser Position aus dem Knochenkanal herausgezogen, wobei sich der Schneidkörper durch die Nut bewegt.

Bevorzugt ist vorgesehen, dass der Schneidkörper nicht direkt an das distale Ende des Bohrers angrenzt, sondern von diesem Ende beabstandet ist, so dass distalseitig des Schneidkörpers ein Endbereich des Schaftes vorsteht. Anseinem distalen Ende weist der Schaft vorzugsweise eine atraumatische Abrundung auf. Diese vereinfacht bei dem Vorhandensein einer Vorbohrung die Positionierung des Bohrers in der Vorbohrung und ermöglicht eine einfachere Zentrierung des Bohrers in dieser Vorbohrung.

Eine vorteilhafte Maßnahme, die Durchführung des Bohrers durch den Knochen zu erleichtern, besteht darin, dass sich eine radiale Außenseite des Schneidkörpers in distaler Richtung vorzugsweise keilförmig verjüngt. Dementsprechend können die die radiale Außenseite des Schneidkörpers begrenzenden Seitenflächen des Schneidkörpers derart ausgerichtet sein, dass sie ausgehend von dem proximalseitigen Ende des Schneidkörpers, an dem dessen Schneide ausgebildet ist, zumindest dort, wo sie an die radiale Außenseite des Schneidkörpers angrenzen, in distaler Richtung spitz aufeinander zu laufen, so dass die radiale Außenseite des Schneidkörpers die Form eines spitzen Dreiecks aufweist. Des Weiteren kann eine distale Stirnseite des Schneidkörpers, die eine Schneide zum Räumen der Nut bildet, in radialer Richtung nach außen in Form eines Dreiecks spitz zulaufen.

Vorteilhafterweise kann an dem Schaft in Achsrichtung des Schaftes und in Verlängerung des Schneidkörpers eine Längsmarkierung ausgebildet sein. Bei dieser Markierung kann es sich z.B. um eine gerade Längseinkerbung an dem Schaft oder um einen auf den Schaft aufgetragenen farbigen Längsstrich handeln. Aufgrund der an dem Schaft ausgebildeten Längsmarkierung ist dem Benutzer des erfindungsgemäßen Bohrers die Drehstellung des Schneidkörpers auch dann erkennbar, wenn sich dieser für den Benutzer nicht sichtbar beispielsweise im Körperinneren in einem Gelenkspalt befindet. Dies ist insofern vorteilhaft, als der Bohrer nach einer retrograden Stufenbohrung nur dann wieder aus dem Knochen herausgezogen werden kann, wenn der Schneidköper mit dem Lichtraumprofil der von dem Schneidkörper erzeugten Nut fluchtet. Insofern muss dem Benutzer nur die Stellung des Schneidkörpers beim anfänglichen Stemmen des Bohrers in den Knochen bekannt sein. Zweckmäßigerweise erstreckt sich die Längsmarkierung parallel zur Längsachse des Schaftes an derselben Winkelposition wie der Schneidkörper. So muss der Benutzer zum Herausziehen des Bohrers lediglich die Längsmarkierung in eine Position gegenüberliegend der Nut bringen, wobei dann automatisch der Schneidkörper mit der Nut fluchtet.

Weiter vorteilhaft kann an dem Schaft proximalseitig des Schneidkörpers eine in Längsrichtung des Schafts verlaufende Längenskala ausgebildet sein. Demzufolge können an dem Schaft in einem Bereich zwischen dessen proximalen Ende und dem Schneidkörper zweckmäßigerweise in gleichen Abständen hintereinander mehrere Markierungen ausgebildet sein, die z.B. quer zur Längsausdehnung des Schafts um dessen Umfang verlaufen. Diese Skala bzw. Markierungen ermöglichen vorteilhafterweise eine Tiefenbestimmung der erzeugten Stufenbohrung mit dem Bohrer selbst.

An dem Bohrer kann im Bereich des distalen Endes des Schafts eine Ausnehmung zur Aufnahme des Schneidkörpers ausgebildet sein. Diese Ausnehmung ist vorzugsweise nutförmig ausgebildet, wobei die Form und Abmessungen der Ausnehmung mit der Geometrie des in die Ausnehmung einzuführenden Bereichs des Schneidkörpers korrespondieren. Vorteilhaft kann der Schneidkörper in der Ausnehmung lösbar befestigt sein, so dass der Schneidkörper bei Bedarf ausgetauscht werden kann. Diese Ausgestaltung ermöglicht es, dass der erfindungsgemäße Bohrer bei Wahl geeigneter Schneidkörper bzw. durch deren Austausch ggf. für unterschiedliche Bohraufgaben eingesetzt werden kann. Ferner ist ein Ersatz des Schneidkörpers bei Verschleiß möglich. Bevorzugt ist allerdings vorgesehen, dass der erfindungsgemäße Bohrer einteilig ausgebildet ist, d.h., der Schneidkörper ein integraler Bestandteil des Bohrers ist.

Wie bereits angemerkt worden ist, ist es zweckmäßig, vor dem Einsatz des erfindungsgemäßen Bohrers eine Vorbohrung vorzunehmen, deren Durchmesser mit dem Durchmesser des zu schaffenden Knochenkanals in dessen nicht erweiterten Bereich übereinstimmt. Vorteilhafterweise entspricht der Außendurchmesser des Schafts im Wesentlichen dem Innendurchmesser dieser vor Einsatz des Bohrers auszuführenden Vorbohrung oder ist geringfügig kleiner. Auf diese Weise ist der Schaft des erfindungsgemäßen Bohrers bei der Schaffung des erweiterten Bereichs des Knochenkanals in der Vorbohrung in Art einer Wellenführung vorzugsweise mit geringem Spiel geführt, was eine exakte Ausrichtung und Ausgestaltung des erweiterten Bereichs des Knochenkanals ermöglicht.

Weiter bevorzugt ist vorgesehen, dass der Schneidkörper an dem Schaft seitlich auskragt. Dementsprechend steht der Schneidkörper und dessen Schneide vorzugsweise mindestens 0,5 mm in radialer Richtung gegenüber der Mantelfläche des Schaftes hervor, so dass Stufenbohrungen geschaffen werden können, bei denen der erweiterte Abschnitt des Knochenkanals einen Durchmesser aufweist, der mindestens 1 mm größer als der übrige Abschnitt des Knochenkanals ist.

Der erfindungsgemäße Bohrer wird zweckmäßigerweise von einer Bohrmaschine angetrieben. Zum Befestigen des Bohrers an der Bohrmaschine weist der Bohrer vorteilhaft an seinem proximalen Ende eine Aufnahme auf. Diese Aufnahme schließt sich proximal an den Schaft an, wobei bevorzugt ein Bauteil sowohl den Schaft als auch die Aufnahme bildet.

Der erfindungsgemäße medizinische Bohrer kann grundsätzlich überall dort eingesetzt werden, wo Stufenbohrungen auszubilden sind. Besonders vorteilhaft ist er aber zum Ausbilden einer Stufenbohrung in einem Knochen und insbesondere für eine Rekonstruktion des vorderen Kreuzbands ausgebildet. Bei einer solchen Rekonstruktion können mit dem erfindungsgemäßen Bohrer in schneller und einfacher Weise an Schienbein und Oberschenkel solche Knochenkanäle zum Festlegen eines Kreuzband-Transplantats geschaffen werden, die jeweils in einem an den Gelenkspalt des Kniekehlgelenks angrenzenden Abschnitt einen erweiterten Durchmesser aufweisen.

Nachfolgend ist die Erfindung anhand eines in den Zeichnungen dargestellten Ausführungsbeispiels näher erläutert. In den Zeichnungen zeigt:

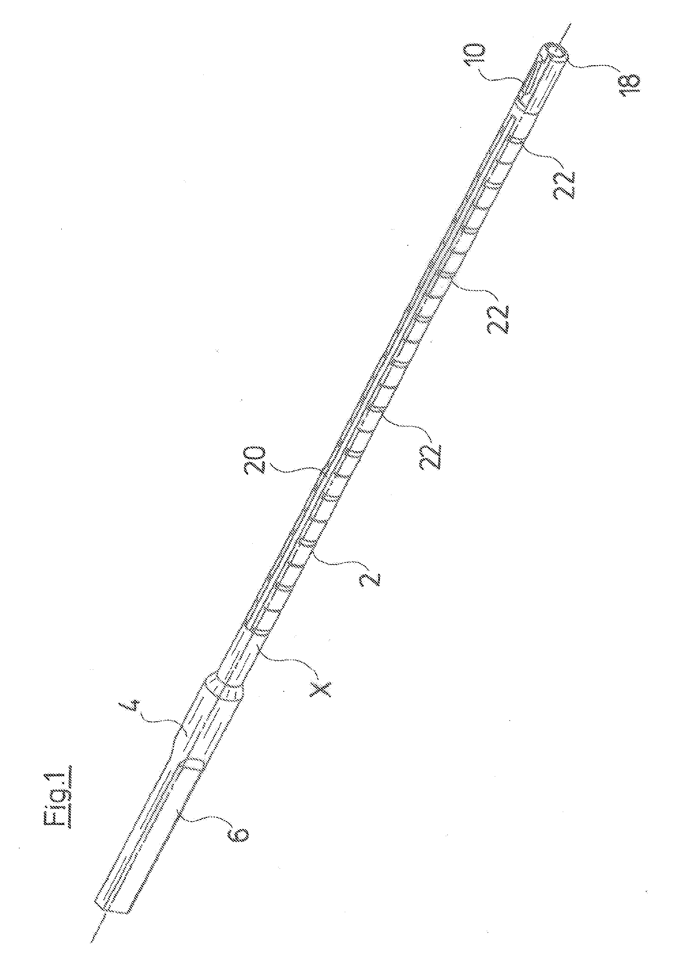

- Fig. 1 -: in perspektivischer Seitenansicht einen medizinischen Bohrer,

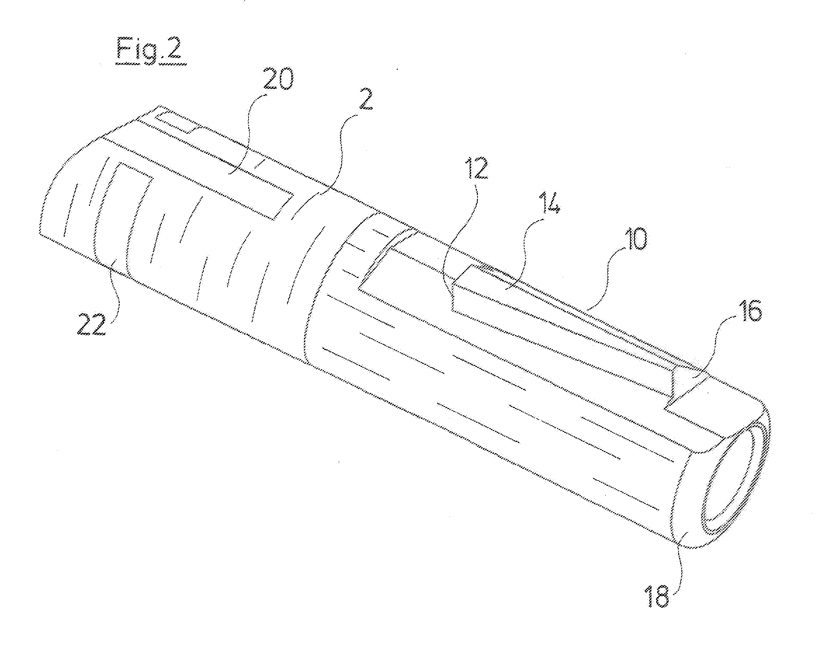

- Fig. 2 -: in vergrößerter perspektivischer Darstellung einen distalen Endabschnitt des Bohrers nach Figur 1 und
- Fig. 3 -: in stark vereinfachter schematischer Darstellung einen mit dem Bohrer zu schaffenden Knochenkanal.

Der dargestellte medizinische Bohrer weist einen zylindrischen Schaft 2 auf. Proximalseitig schließt sich an den Schaft 2 eine Aufnahme 4 zum Einspannen des Bohrers in ein Spannfutter einer Bohrmaschine an.

An einem in Richtung des proximalen Endes des Bohrers weisenden Ende des Schneidkörpers 10 ist eine Schneide 12 ausgebildet. Eine radiale Außenseite 14 des Schneidkörpers 10 spitzt sich in distaler Richtung keilförmig zu. Eine distale Stirnseite 16 des Schneidkörpers 10, die senkrecht zur Längsachse X des Schalters 2 angeordnet ist, spitzt sich in radialer Richtung in Form eines Dreiecks zu.

Der Schneidkörper 10 ist nicht direkt an dem distalen Ende des Bohrers angeordnet, so dass ein Bereich des Schaftes 2 distalseitig des Schneidkörpers 10 hervorsteht. Dieser Bereich des Schaftes 2 ist stirnseitig mit einem Radius 18 versehen und bildet so eine atraumatische Spitze 18 des Bohrers.

In einem Bereich zwischen dem Schneidkörper 10 und der an den Schaft 2 angrenzenden Aufnahme 4 ist der Schaft 2 mit einer Längsmarkierung 20 versehen. Bei der Längsmarkierung 20 handelt es sich um einen geraden farbigen Strich und/oder um eine Vertiefung, welche(r) in Verlängerung des Schneidkörpers 10 angeordnet und ausgerichtet ist und parallel zur Längsachse X verläuft. Die Längsmarkierung 20 dient zur Bestimmung der Winkellage des Schneidkörpers 10 während des Einsatzes des Bohrers im Körperinneren.

Darüber hinaus ist an dem Schaft 2 des Bohrers in einem Bereich distalseitig des Schneidkörpers 10 eine Längenskala ausgebildet. Diese Längenskala besteht aus mehreren in Umfangsrichtung des Schaftes 2 verlaufenden Markierungen 22, die in Längsrichtung des Schaftes 2 in gleichen Abständen hintereinander angeordnet sind. Die Längenskala ermöglicht eine Bestimmung der Bohrtiefe.

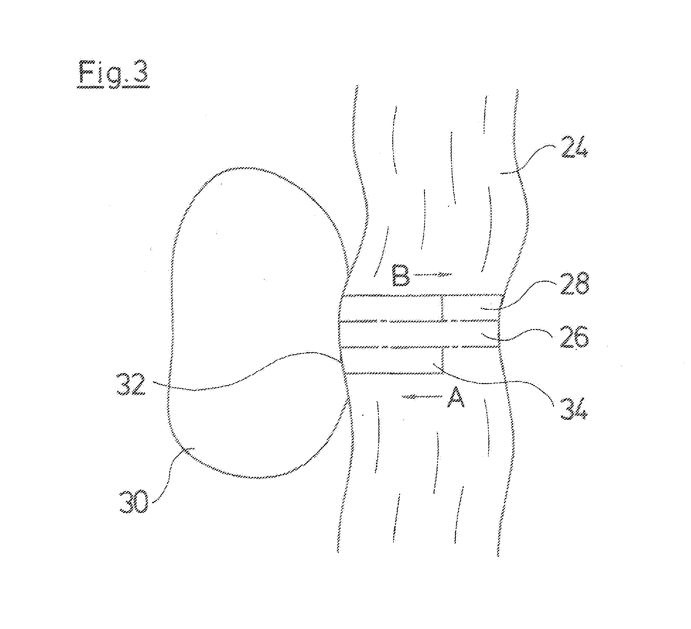

Die Vorgehensweise beim Bohren einer Stufenbohrung (Fig. 3) mit dem dorgstellten Bohrer ist wie folgt:

Zunächst wird der Bohrer in einer reinen Linearbewegung, d.h. ohne Bohrerdrehung in den Knochen 24 gestoßen. Um diesen Vorgang zu erleichtern kann zweckmäßigerweise zuvor eine durch den Knochen 24 führende Vorbohrung 26 (in Fig. 3 strichpunktiert dargestellt) vorgenommen werden, deren Durchmesser dem nicht erweiterten Bereich des zu schaffenden Knochenkanals und dem Außendurchmesser des Schaftes 2 entspricht. Das Ansetzen des Bohrers in der Vorbohrung 26 erleichtert die an dem distalen Ende des Bohrers ausgebildete Spitze 18. Hierbei wird zweckmäßigerweise darauf geachtet, dass sich der Schneidkörper 10 bzw. die Längsmarkierung 20 in einer prägnanten Position beispielsweise einer Zwölf-Uhr-Position befindet.

Beim geraden Durchstoßen der Vorbohrung 26 an dem Knochen 24 bildet die Stirnseite 16 des Schneidkörpers 10 eine Schneide, mit der durch Räumen an die Vorbohrung 26 angrenzend eine Längsnut 28 ausgeschnitten wird. Nach dem Durchstoßen des Knochens 24, wenn sich der Schneidkörper 10 außerhalb des Knochens 24 beispielsweise in einem dahinter befindlichen Hohlraum 30 befindet, wird der Bohrer drehend angetrieben und entgegen der vormaligen Stossrichtung A in Richtung B zurückgezogen. Hierbei kommt die an dem Schneidkörper 10 ausgebildete Schneide 12 an dem Rand 32 der Vorbohrung 26 in Kontakt und es wird mit der Schneide 12 ein erweiterter Bereich 34 des zu schaffenden Knochenkanals ausgeschnitten. Hierbei kann der Benutzer des Bohrers die Bohrtiefe beim Aufbohren des erweiterten Bereichs 34 mittels der an dem Schaft 2 ausgebildeten Längenskala überwachen. Ist die Bohrtiefe ausreichend, wird der Antrieb des Bohrers abgeschaltet. Nun kann der Bohrer manuell in eine Stellung bewegt werden, in der sich der Schneidkörper 10 bzw. die Längsmarkierung 20 in einer Stellung befindet, die derjenigen vor dem Durchstoßen der Vorbohrung 26 entspricht. In diesem Fall ist sichergestellt, dass sich der radial auskragende Schneidkörper 10 innerhalb des Lichtraumprofils der an der Vorbohrung 26 angrenzenden Längsnut 28 befindet, so dass der Bohrer problemlos aus der Vorbohrung 26 herausgezogen werden kann. Der Bohrvorgang ist damit abgeschlossen.

### Bezugszeichenliste

- 2 -: Schaft
- 4 -: Aufnahme
- 6 -: Flachseite
- 10 -: Schneidkörper
- 12 -: Schneide
- 14 -: Außenseite
- 16 -: Stirnseite
- 18 -: Radius
- 20 -: Längsmarkierung
- 22 -: Markierung
- 24 -: Knochen
- 26 -: Vorbohrung
- 28 -: Längsnut
- 30 -: Hohlraum
- 32 -: Rand
- 34 -: Bereich
- A -: Stossrichtung
- B -: Richtung
- X -: Längsachse

## Patentansprüche

1. Medizinischer Bohrer mit einem Schaft (2) und mit einem im Bereich eines distalen Endes des Schafts (2) feststehend angeordneten Schneidkörper (10), welcher radial an dem Schaft (2) auskragt und welcher eine einem proximalen Ende des Schaftes (2) zugewandte Schneide (12) aufweist.

2. Medizinischer Bohrer nach Anspruch 1, bei dem sich das distale Ende des Schaftes (2) distalseitig des Schneidkörpers (10) zu einer Spitze (16) verjüngt.

3. Medizinischer Bohrer nach einem der vorangehenden Ansprüche, bei dem sich eine radiale Außenseite (14) des Schneidkörpers (10) in distaler Richtung vorzugsweise keilförmig verjüngt.

4. Medizinischer Bohrer nach einem der vorangehenden Ansprüche, bei dem an dem Schaft (2) in Achsrichtung des Schafts (2) und in Verlängerung des Schneidkörpers (10) eine Längsmarkierung (20) ausgebildet ist.

5. Medizinischer Bohrer nach einem der vorangehenden Ansprüche, bei dem an dem Schaft (2) proximalseitig des Schneidkörpers (10) eine in Längsrichtung des Schafts (2) verlaufende Längenskala (22) ausgebildet ist.

6. Medizinischer Bohrer nach einem der vorangehenden Ansprüche, bei dem der Außendurchmesser des Schafts (2) dem Innendurchmesser einer vor Einsatz des Bohrers auszuführenden Vorbohrung (26) entspricht.

7. Medizinischer Bohrer nach einem der vorangehenden Ansprüche, welcher am proximalen Ende eine Aufnahme (4) für eine Bohrmaschine aufweist.

8. Medizinischer Bohrer nach einem der vorangehenden Ansprüche, welcher zum Ausbilden einer Stufenbohrung in einem Knochen (24), insbesondere für eine Kreuzband-Rekonstruktion ausgebildet ist.
